# EUROPEAN PATENT APPLICATION

(11) **EP 4 276 626 A1**
(43) Date of publication of application: **15.11.2023**
(21) Application number: 22173201.9
(22) Date of filing: 13.05.2022
(51) Int. Cl.: G06F 11/00, G16H 40/40, G06F 16/00, G05B 23/02, G06F 11/34, G06F 16/34, G06F 17/40

(54) **MEDICAL IMAGING DEVICE FAULT RESOLUTION**

(71) Applicant: Koninklijke Philips N.V., 5656 AG Eindhoven (NL)
(72) Inventor: NAIK, Sarif Kumar, Eindhoven (NL); ANAND, Shreya, Eindhoven (NL); PAUL, Soubhik, Eindhoven (NL)
(74) Representative: Philips Intellectual Property & Standards

(57) **Abstract**

A method for identifying a log file for resolution of a fault of a medical imaging device, is provided. The method includes: obtaining log file browsing data describing one or more log files of the medical imaging device already viewed by a user to resolve the fault of the medical imaging device; obtaining problem data describing the fault of the medical imaging device; inputting the problem data and the log file browsing data to a machine learning algorithm, the machine learning algorithm being trained to predict, for each of a plurality of log files of the medical imaging device, and based on the browsing data, a resolution probability indicating a likelihood that the log file will assist in resolution of the fault of the medical imaging device; obtaining a prediction result from the machine learning algorithm in response to the inputting, the prediction result comprising a resolution probability for one or more of the plurality of log files of the medical imaging device; and identifying a log file for resolution of the fault of the medical imaging device based on the obtained prediction result.

## Description

### FIELD OF THE INVENTION

The present invention relates to the field of equipment maintenance, and in particular to the field of resolving faults of a medical imaging device.

### BACKGROUND OF THE INVENTION

A medical imaging device may occasionally malfunction, i.e., exhibit a fault and fail to work properly.

Typically, in response to a fault in a medical imaging device, one or multiple service engineers are assigned to diagnose and resolve the fault. Such service engineers may perform the diagnosis remotely, or on site.

During fault diagnosis, the service engineer(s) may need to inspect the log files of the medical imaging device to identify relevant diagnostic information such as error messages. The log files of a medical imaging device may contain critical information which helps service engineers identify the root cause of the fault.

Log files may be retrieved on-demand so that they contain the most recent information about the device. However, due to data size and number of concurrent requests, it can take a significant amount of time to download and search through log files. The resultant time delay impacts troubleshooting and causes additional system downtime.

There is therefore a desire to reduce the length of time taken to identify the cause or solution to a fault in a medical imaging device.

### SUMMARY OF THE INVENTION

The invention is defined by the claims.

According to examples in accordance with an aspect of the invention, there is provided a method for identifying a log file for resolution of a fault of a medical imaging device, the method comprising:
obtaining log file browsing data describing one or more log files of the medical imaging device already viewed by a user to resolve the fault of the medical imaging device;
obtaining problem data describing the fault of the medical imaging device;
inputting the problem data and the log file browsing data to a machine learning algorithm, the machine learning algorithm being trained to predict, for each of a plurality of log files of the medical imaging device, and based on the browsing data, a resolution probability indicating a likelihood that the log file will assist in resolution of the fault of the medical imaging device;
obtaining a prediction result from the machine learning algorithm in response to the inputting, the prediction result comprising a resolution probability for one or more of the plurality of log files of the medical imaging device; and
identifying a log file for resolution of the fault of the medical imaging device based on the obtained prediction result.

Proposed concepts thus aim to provide schemes, solutions, concepts, designs, methods and systems pertaining to aiding fault resolution through the consideration of the likelihood that the log file will assist in resolution of the fault.

In particular, embodiments of the invention propose generating predicted resolution probabilities for different log files based on the log file browsing data describing one or more log files of the medical imaging device already viewed by a user to resolve the fault of the medical imaging device, or in other words, a user's browsing history of log files. The most relevant or useful log file(s) for a user to look at next may then be suggested or prioritized for provision to an engineer, for example.

A proposed concept recognizes that different log files may be of different relevance to a particular fault, and that such relevance may be identified in order to assist in the exploration and/or selection of log files for fault diagnosis. It is also proposed that the relevance of log files may be learnt (e.g. from historical log browsing data) and thus predicted using machine-learning.

Embodiments may therefore simplify the job of a service engineer who wants to explore the log files for diagnosing a fault, by assisting the exploration of different log files in a guided and predictive manner. Such embodiments may provide the advantages of reduced search/exploration time and with improved diagnostic accuracy. For instance, embodiment may enables a service engineer to select a most appropriate next log file when searching log files, thus enabling irrelevant log files to be ignored (i.e. prevented from provision to the engineer). This may also avoid unnecessary data retrieval/downloading. By way of further example, when browsing through log files, a service engineer may be provided with a suggestion of one or more log files to log at next, and thus be guided along a browsing that is deemed optimal for fault resolution.

In other words, embodiments propose to use machine learning to generate predictions of the likelihood that log files will assist in resolution of a fault of the medical imaging device. Such predictions may be based on a user's browsing history of log files, thus accounting for a preceding log file searching/browsing route taken by the user, and facilitating the suggestion of which log file(s) to look at next (i.e. guiding the next step(s) of a user's log file search/exploration). Predictions provided by embodiment may thus aid the prioritisation of log files for fault analysis and/or diagnosis. Accordingly, embodiments may be used in relation to fault diagnosis so as support a service engineer when diagnosing and resolving the fault. Improved (e.g. quicker) fault resolution in medical imaging devices may therefore be provided by proposed concepts.

The machine learning algorithm may be trained using a training algorithm configured to receive an array of training inputs and respective known outputs, wherein a training input comprises: problem data; and log file browsing data, and wherein a respective known output comprises, for each of a plurality of log files of the medical imaging device, a resolution probability indicating a likelihood that the log file will assist in resolution of the fault of the medical imaging device based on the browsing data. In this way, the machine learning algorithm may be trained to output a resolution probability prediction for a log file when provided with log file browsing data and a description of the fault of the medical imaging device. Previous log file browsing/searching routes that led to fault resolution may thus be learnt and leveraged to make future suggestions for browsing/searching log files when faced with the same (or similar) problems/faults. For instance, a respective known output may further comprise a description of solution associated with the problem data. In this way, previously-obtained solutions may be used for reference/training along with the browsing data for a similar case in the future.

In some embodiments, obtaining log file browsing data may comprise: monitoring access to the plurality of log files of the medical imaging device of file access; and generating log file browsing data based on the result of monitoring. In this way, the log file browsing actions and steps of a user may be automatically monitored and recorded so as to provide data for use with the machine learning algorithm. A need for a service engineer to actively record and/or provide information about previous log file interaction(s) may therefore be avoided/alleviated by proposed embodiments.

The process of obtaining log file browsing data may comprise analysing a usage history of a log file viewer application. That is, embodiments may take advantage of functionality of a log file viewer application to obtain information about a user's previous access to the log files. Existing/conventional log file viewer applications may therefore be leveraged to facilitate the implementation of proposed embodiments.

In some embodiments, obtaining problem input data may comprise: receiving, via in input interface, problem data provided by a respondent in response to a request for data. The request for data may, for example, comprise a fault analysis questionnaire, or from old maintenance records commented in earlier similar failure. In this way, responses of the respondent (e.g. end user or service engineer) to the questionnaire may be provided as problem input data. Such responses may thus be used to identify one or more features of the fault. Embodiments may therefore cater for natural language description of a medical imaging device fault, thereby facilitating simple and intuitive data provision.

In an embodiment, the step of obtaining problem data describing the fault of the medical imaging device may comprise performing a natural language processing analysis on a description the fault of the medical imaging device.

Some embodiments may further comprise the step of determining a data visualisation method based on the obtained prediction result. This may, for example, involve parameters of associated with the identified log file for resolution of the fault, thereby ensuring that a type of data involved in the log file may be visualized/displayed in an optimal or appropriate manner. For instance, if the next step is to look at the temperature of a parameter, then it is a numeric value over a period in Celsius or Fahrenheit. A time series plot can be created and shown along with a regression line or other statistical measure.

Some embodiments may further comprise the step of transmitting the identified log file over a communication network. Advantageously, since the log file that is transmitted has been identified in the manner described herein, the amount of data that is transmitted from the medical imaging device in order to resolve the fault of the medical imaging device is reduced. This overcomes bandwidth limitations in transmitting large log files across a bandwidth-constrained communication network.

Some embodiments may further comprise the steps of analysing the identified log file to provide an indication of the fault of the medical imaging device, and optionally adjusting one or more operational parameters of the medical imaging device to resolve the fault of the medical imaging device. The identified log file may be analysed using known analysis techniques. The fault may then be rectified manually, or automatically. For instance, in some embodiments, the operational parameters of the medical imaging device may be automatically adjusted in order to rectify the fault.

In some embodiments, the machine learning algorithm may comprise a graph neural network.

According to another aspect of the invention, there is provided a method for resolving a fault of a medical imaging device, the method comprising:
identifying a log file for resolution of the fault of the medical imaging device according to the method of a proposed embodiment;
generating updated log file browsing data based on the identified log file;
inputting the problem data and the updated log file browsing data to the machine learning algorithm;
obtaining an updated prediction result from the machine learning algorithm in response to the inputting, the updated prediction result comprising a resolution probability for one or more of the log files of the medical imaging device; and
identifying a second log file for resolution of the fault of the medical imaging device based on the obtained prediction result.

A concept for guiding a user along a log file browsing route towards a solution to a fault of a medical imaging device may therefore be proposed, and this may then be used to prioritize the searching and/or provision of log files to an engineer for example.

According to another aspect, there is provided a computer program product, wherein the computer program product comprises a computer-readable storage medium having computer-readable program code embodied therewith, the computer-readable program code configured to perform all of the steps of a proposed embodiment.

Thus, there may also be provided a computer system comprising: a computer program product according to proposed embodiment; and one or more processors adapted to perform a method according to a proposed concept by execution of the computer-readable program code of said computer program product.

According to still another aspect of the invention, there is provided a system for identifying a log file for resolution of a fault of a medical imaging device, the system comprising:
a data interface configured to obtain: log file browsing data describing one or more log files of the medical imaging device already viewed by a user to resolve the fault of the medical imaging device; and problem data describing the fault of the medical imaging device;
a machine learning algorithm configured to receive the problem data and the log file browsing data, the machine learning algorithm being trained to output a prediction result comprising a resolution probability for each of a plurality of log files of the medical imaging device, the resolution probability indicating a likelihood that the log file will assist in resolution of the fault of the medical imaging device based on the browsing data; and
a processor configured to identify a log file for resolution of the fault of the medical imaging device based on the obtained prediction result.

The system may be remotely located from a user device for analyzing and/or diagnosing a medical imaging device fault. In this way, a user (such as a service engineer) may have an appropriately arranged system that can receive information about the relevance and/or usefulness of log files at a location remotely located from the system. Embodiments may therefore enable a user to dynamically adjust or adapt log file browsing and/or retrieval using a local system (which may, for example, comprise a portable display device, such as a laptop, tablet computer, mobile phone, PDA, etc.). By way of example, embodiments may provide an application for a mobile computing device, and the application may be executed and/or controlled by a user of the mobile computing device.

The system may further include: a server device comprising the system for identifying a log file for resolution of a fault of a medical imaging device; and a client device comprising a userinterface. Dedicated data processing means may therefore be employed for the purpose of identifying (150) a log file for resolution of a fault of a medical imaging device, thus reducing processing requirements or capabilities of other components or devices of the system.

The system may further include a client device, wherein the client device comprises the data interface, machine learning algorithm, processor, and a display unit. In other words, a user (such as a service engineer) may have an appropriately arranged client device (such as a laptop, tablet computer, mobile phone, PDA, etc.) which processes received data in order to identify a log file for resolution of a fault and generate a display control signal. Purely by way of example, embodiments may therefore provide a log file analysis system that enables prioritization of log files, wherein real-time communication between a user (e.g. service engineer) and a medical imaging device is provided and can have its functionality extended or modified according to proposed concepts, for example.

It will be understood that processing capabilities may therefore be distributed throughout the system in different ways according to predetermined constraints and/or availability of processing resources.

One or more concepts are provided for optimizing a search or review of log files for medical image device fault diagnostics or resolution. By way of example, proposed embodiments may address the issue of excessive log file searching and/or waiting time for an engineer to receive log files. Time spent reviewing or downloading the irrelevant (or less relevant) log files may be reduced or minimized.

These and other aspects of the invention will be apparent from and elucidated with reference to the embodiment(s) described hereinafter.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a flow diagram of a method for method for identifying a log file for resolution of a fault of a medical imaging device according to an embodiment;
Fig. 2 is a flow diagram illustrating the use of the method of Fig. 1 in a method for resolving a fault of a medical imaging device according to an embodiment;
Fig. 3A illustrates the use of shortest path first algorithm for first and second browsing graphs;
Fig. 3B illustrates how an optimized path within a browsing graph can be derived to identify the shortest browsing trail;
Fig. 4 illustrates an exemplary embodiment for predicting a browsing path and next best step;
Fig. 5 depicts an exemplary structure of a GNN that may be employed by each of the models of Fig. 4;
Fig. 6 depicts a simplified block diagram of a system for identifying a log file for resolution of a fault of a medical imaging device according to an embodiment; and
Fig. 7 is a simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

The invention proposes concepts for aiding fault diagnosis of a medical imaging device. In particular, embodiments may provide a method and/or system which takes account of a user's browsing history of log files and then identifies (e.g. suggests) a log file for resolution of the fault at hand. In this way, as an engineer searches/browses through log files in order to diagnose/solve a fault, one or more log files to review next may be suggested to the engineer.

Proposed concepts may be based on a realisation that different service engineers may take different routes to diagnose or solve faults. The time taken to arrive at a solution may depend on the browsing/searching route taken, with some routes potentially not being optimal. Using machine learning (trained using previous log file searching/browsing routes to solutions), the likelihood that log files and/or content provided therein may assist in the resolution of a fault may be predicted. A 'best next log file' may therefore be identified and suggested to a user, according to the log file(s) already reviewed by the user. In this way, irrelevant log files may therefore be identified and de-prioritised, thus reducing unnecessary data review and/or retrieval. For instance, embodiments may facilitate the identification and prioritisation of the next most useful log file to review when searching through log files to resolve a particular fault, thus enabling an engineer to be guided through a log file browsing route that may be optimal for resolving a fault. The review process is often performed remotely via a communication network. Moreover, the log files typically include very large amount of data. Consequently, the retrieval of log files for review at a remote location can result in bandwidth issues for the communication network. Sometimes the transfer of log files for analysis is delayed until a quieter period when sufficient bandwidth is available, or the risk of interrupting other vital communications is reduced, which delays resolution of a fault. By identifying and prioritising the next most useful log file to review, these bandwidth issues may be alleviated by reducing the number of irrelevant log files that are retrieved, and thus transmitted via the communication network.

Accordingly, embodiments may be used in relation to medical imaging fault analysis and/or diagnosis.

Also proposed is a concept for resolving a fault of a medical imaging device. This may be used to guide the provision of log files to an engineer.

Proposed embodiments may thus facilitate improved (e.g. quicker and/or easier) fault analysis and/or diagnosis through the improved identification of relevant information to an engineer. Embodiments of the present invention are therefore directed toward enabling the improved searching of log files for fault diagnosis of a medical imaging device. Embodiment may provide an efficient and predictive log file exploration mechanism that enables prediction of a 'next step' (i.e. next log file suggestion) at each step during log browsing. Embodiments may also facilitate prediction of best visualization method, line/bar chart, distribution plots, tables, etc. A reduction in a time to diagnose a fault/problem may therefore be provided by proposed embodiments.

By way of example only, illustrative embodiments may be utilized in many different types of clinical, medical or subject-related environments, such as a hospital, doctor's office, ward, care home, person's home, etc. Illustrative embodiments may, for example, be employed in digital pathology platforms as a clinical decision support system.

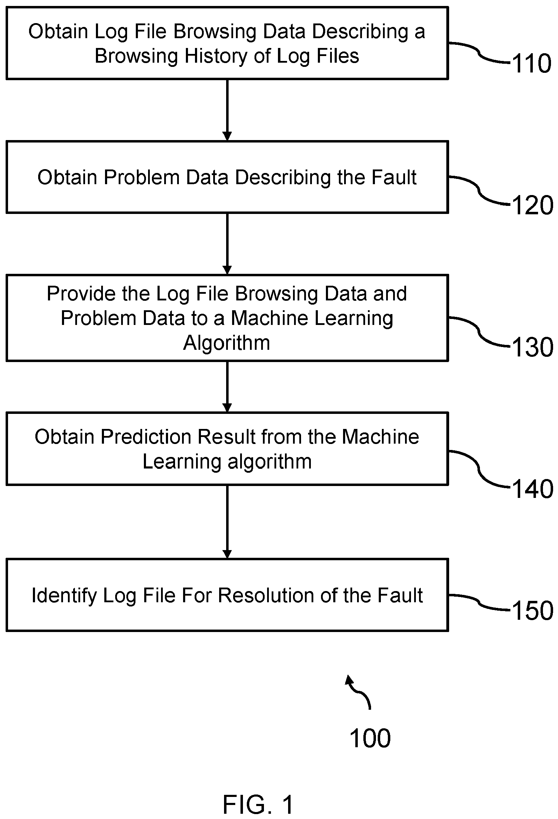

Fig. 1 illustrates an embodiment of a computer-implemented method 100 for identifying a log file for resolution of a fault of a medical imaging device. The log file to be identified is a suggestion of the next log file for a user to review according to: (i) the fault at hand; and (ii) the preceding log files that have been reviewed (i.e. preceding browsing activity of the user).

The method begins with step 110 of obtaining log file browsing data describing one or more log files of the medical imaging device already viewed by a user to resolve the fault of the medical imaging device. Here, step 110 of obtaining initial problem input data comprises: monitoring access to the plurality of log files of the medical imaging device of file access; and generating log file browsing data based on the result of monitoring.

However, in other embodiments, step 110 of obtaining initial problem input data may comprise analyzing a usage history of a log file viewer application. For instance, such embodiments may leverage functionality (e.g. an activity log/record) of a log file viewer application to obtain information about the user's previous access to the log files.

The method then proceeds to step 120 of obtaining problem data describing the fault of the medical imaging device. Specifically, the step 120 of obtaining problem input data comprises: receiving, via in input interface, problem data provided by a respondent in response to a request for data. In particular, the request for data comprises a fault analysis questionnaire provided to an engineer (e.g. via a user interface). Responses of the engineer to the questions of the questionnaire are thus obtained as initial problem input data and used to identify one or more features of the fault. Step 120 may also comprise performing a natural language processing (NLP) analysis on the problem data to identify one or more features of the medical imaging device fault. The NLP analysis processes natural language description(s) of the fault, thereby facilitating the identification of features/aspects of the fault from information provided in a simple and intuitive manner.

At step 130, the problem data and the log file browsing data are provided to a neural network (NN)-based machine learning algorithm. The machine learning algorithm is trained to predict, for each of a plurality of log files of the medical imaging device, and based on the browsing data, a resolution probability indicating a likelihood that the log file will assist in resolution of the fault of the medical imaging device.

The structure of an artificial NN (or, simply, neural network (NN)) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

There are several types of neural network, such as convolutional neural networks (CNNs) and recurrent neural networks (RNNs). However, the exemplary embodiment of Fig. 1 employs a Graph Neural Network (GNN). For a given graph (with respect to a log file browsing trail), the GNN model can predict the link with the next best step which is one of the candidate node. The GNN models the relationship, context and connections among the browsing trails graph nodes. The model is trained on previous browsing trail graphs and it learns different features of each node, state of the neighbouring node and features of neighbouring node to predict the next best step i.e. link to the best fitted node.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, weightings of the mathematical operation of each neuron may be modified until the error converges. Known methods of modifying a neural network include gradient descent, backpropagation algorithms and so on.

The training input data entries for the machine learning algorithm used in method 100 correspond to example problem data and log file browsing data. The training output data entries correspond to resolution probabilities, for each of a plurality of log files of the medical imaging device, indicating a likelihood that the log file will assist in resolution of the fault of the medical imaging device based on the browsing data. That is, the machine learning algorithm is trained using a training algorithm configured to receive an array of training inputs and respective known outputs, wherein a training input comprises problem data and associated log file browsing data and respective known output comprises, for each of a plurality of log files of the medical imaging device, a resolution probability indicating a likelihood that the log file will assist in resolution of the fault of the medical imaging device. In this way, the machine learning algorithm is trained to output a resolution probability prediction for a log file when provided with a description of a fault of a medical imaging device and one or more log files of the medical imaging device already viewed by a user to resolve the fault of the medical imaging device (e.g. preceding log files reviewed).

At step 130, a prediction result is obtained from the machine learning algorithm in response to the inputting. The prediction result comprises a resolution probability for one or more of the plurality of log files of the medical imaging device.

Finally, at step 140, a log file for resolution of the fault of the medical imaging device is identified based on the obtained prediction result. Specifically, in the exemplary embodiment of Fig. 1, the step of identifying a log file for resolution of the fault comprises determining the log file with the highest predicted resolution probability.

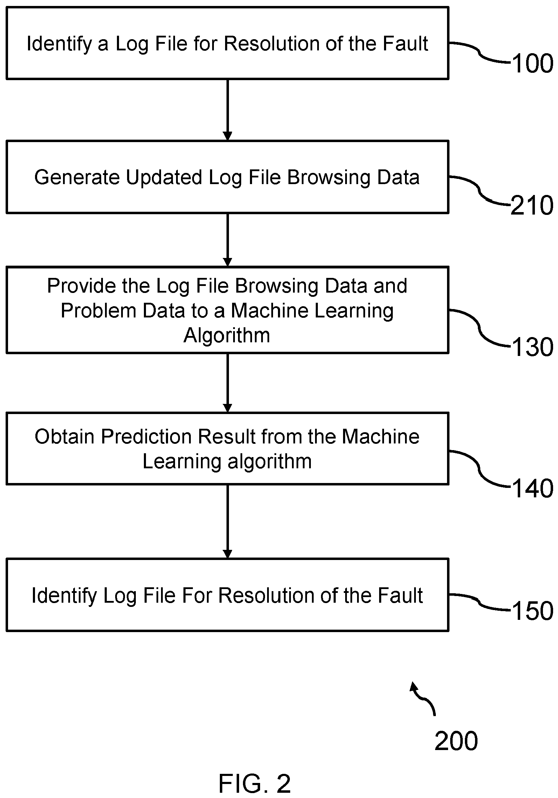

Fig. 2 illustrates the use of the method of Fig. 1 in a computer-implemented method 200 for resolving a fault of a medical imaging device according to an embodiment.

The method 200 begins with implementing the method 100 of Fig. 1 as described above, from which a (first) log file for resolution of the fault is identified. That is, a suggestion for a (first) next log file to review is determined according to a (first) prediction result from the embodiment of Fig. 1.

Next, the method proceeds to step 210 in which updated log file browsing data is generated based on the identified (first) log file. The updated log file browsing data is thus modified to take account of the identified (first) log file being the last (i.e. most recent) log file reviewed by the user.

At step 130, the problem data and the updated log file browsing data are provided to the neural network (NN)-based machine learning algorithm of the embodiment of Fig. 1. As described above, the machine learning algorithm is trained to output a resolution probability prediction for a log file when provided with a description of a fault of a medical imaging device and the updated browsing history of log files (e.g. the preceding log files reviewed).

At step 140, a (second) prediction result is obtained from the machine learning algorithm. The second prediction result comprises a (second/new/updated) resolution probability for one or more of the plurality of log files of the medical imaging device.

Finally, at step 150, a (second) log file for resolution of the fault of the medical imaging device is identified based on the obtained prediction result. That is, a suggestion for a (second) next log file to review is determined according to a (second) prediction result which takes account of the updated log file browsing data.

It will be understood that this method can be repeated, with the log file browsing data being updated to take account of the newly-identified log files. In this way, a browsing route/path can be suggested to the user, with the browsing route/path being predicted to lead the user towards a solution for the fault (taking account of previous routes to solutions learnt by the machine learning algorithm).

By way of further explanation of the proposed concept(s) associated with the embodiments described above, main elements of exemplary embodiments may be summarised as follows:

### (A) Log File Browsing Trail Logging Mechanism

Log files/tables browsed by service engineer may be monitored and recorded. Also, log file browsing steps and browsing pattern of log content may also be detected and recorded. This may also comprise tagging the log files that a service engineer is likely to use for a confirmation of issue. In logging log file browsing history, access parameters and observed patterns can be observed and recorded. The confirmed issue (i.e. fault) may then be associated with the action taken (or part replaced).

### (B) Identify Next Log File (Using Supervised Learning)

Most probable failure mode of failure can be identified (e.g. using existing/known techniques. A service log on the issue can then be reviewed in order to process service comments, estimate the related logs, error patterns, parameters, etc. The next suitable log file(s) to be used can then be predicted using a machine learning algorithm.

### (C) Predict Each Next Step

At each step, the next log file to be viewed may be predicted. In doing so, an estimate of a best visualization method [e.g. descriptive content, tabular, frequency plot, regression lines, etc.] may be generated based on the content to be viewed. This may involve trend analysis.

Any steps deviated from predicted steps (i.e. suggested next log file(s) may be leveraged as a feedback loop for reinforcement learning to improve subsequent predictions.

Each of the above-mentioned elements will now be described in more detail, simply to provide exemplary information and explanation of the proposed concept(s).

### (A) Log File Browsing Trail Logging Mechanism

This may involve the collection of the log file browsing history (i.e. log file browsing data), including log files, tables and other files viewed by the service engineer. The collection of these actions may be thought of as the browsing trail of a particular service engineer.

Generally, a log viewer software/application is used by a service engineer to view log files, navigate log file content, look at different tables/flow charts, etc. The browsing history or browsing trail can thus be derived/detected from the usage history of log viewer software, and different components of browsing trails (like portion(s) of log file viewed, tables viewed, tables queried, error codes viewed, etc.) can be extracted and used to generate log file browsing data. The browsing trail can differ from one service engineer to another, depending on the way he/she approaches a problem/fault at hand.

The point at which the service engineer starts to solve the problem (or execute the solution), the log files which were used to get to the solution may be tagged to the problem statement (i.e. associated with the problem/fault). A problem/fault description (e.g. problem statement) may be tagged to one or many browsing trails, depending on the number of service engineers who have solved the problem/fault in the past.

Each browsing trail can vary in terms of time to solution and also the route/number of browsing steps in the trail. Once the solution to a problem is attained, the solution is also tagged to (i.e. associated with) the problem along with the browsing trail. This information can then be used to train the machine learning algorithm.

### (B) Identify Next Log File (Using Supervised Learning)

For further processing of the browsing trails/paths (for use in identifying failure cause and also prediction of next appropriate step), a log file browsing trail is structured into a machine readable format. An exemplary approach to this is to employ NLP techniques to summarize the log file browsing trails. Various tools for text summarization are known and widely available and may be employed for such a purpose.

By way of example, the following NLP steps can be used for summarization.
(i) Convert paragraphs to sentences;
(ii) Text pre-processing: remove all the special characters, stop words and numbers;
(iii) Tokenize the sentences;
(iv) Find weighted frequency of occurrence;
(v) Replace words by weighted frequency in original sentences;
(vi) Sort sentences in descending order of sum: The sentences with highest frequencies to summarize the text.

Next, directed graphs are derived from these browsing trail components, each user's browsing trails being represented by a corresponding directed graph.

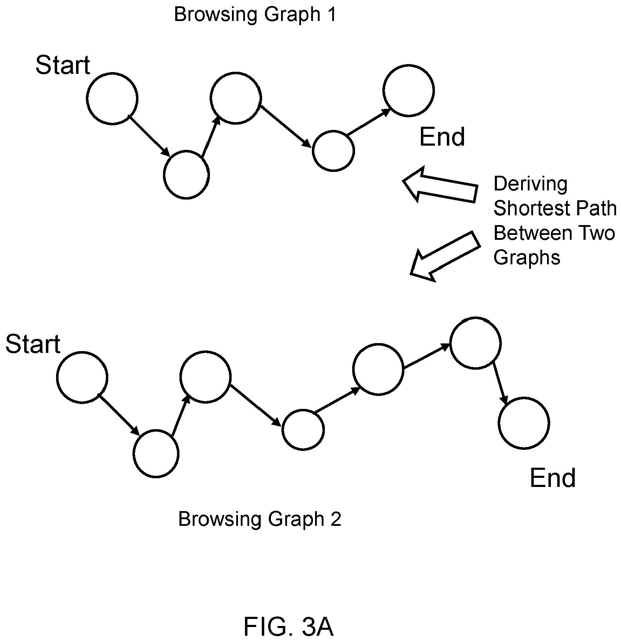

Using standard graph algorithms, such as shortest path first, the optimum browsing trail for a particular issue can be identified (as depicted in Fig. 3A).

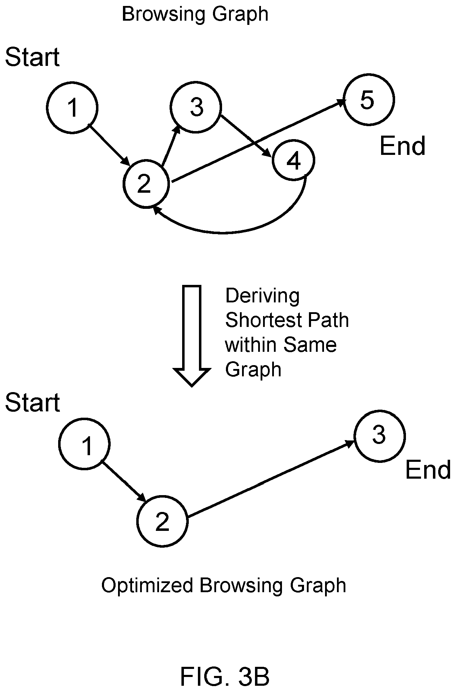

The optimized path within a graph can also be derived to identify the shortest browsing trail (as depicted in Fig. 3B).

The summary of the browsing trail, also known as the augmented logs, are associated (e.g. tagged) with the fault description for further reference and processing. The point where the service engineer starts solving the issue/fault becomes a part of the solution (like replacing a part, fixing a bug, etc.). The solution is also associated with the fault description so that it can be used for future reference along with the browsing trail (e.g. in case similar problems arise in future).

The association approach (e.g. tagging) is similar to Subordinate Clause detection which comprises linking a main problem description to the cause of the problem (based on the log file browsing path/trail). The fault/problem with the system may mostly depend on past/preceding events that are recorded in the log files, similar to following example, e.g. subordinate clause detection:
The door opened, because the man pushed it.

Clause subordination links two clauses so that one, the subordinate clause (SC), is syntactically dependent on and embedded in the other, the main clause. A defining characteristic of a SC is its dependency on another clause, which means that it cannot stand by itself. It has to be embedded into another clause and functions as a constituent of that clause. Hence, the clause "because the man pushed it" is a SC, providing information on the cause of the event described by the main clause (door opening).

### (C) Predict Each Next Step

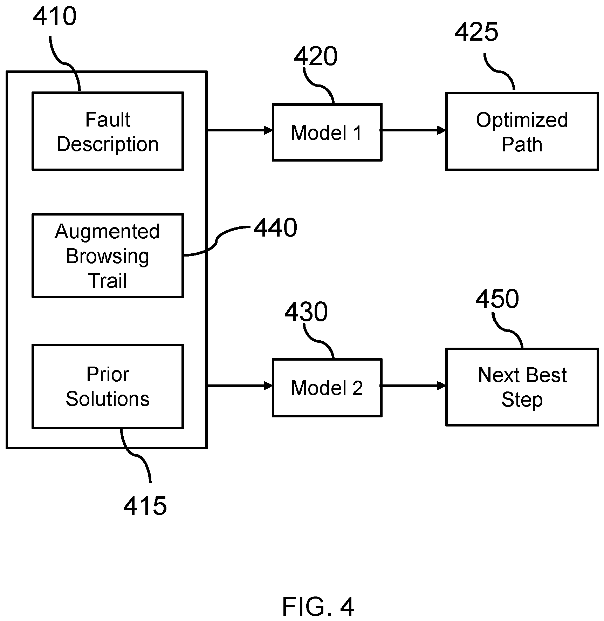

Referring to Fig. 4, the augmented logs (405) that are associated with the fault description, along with new fault description (i.e. problem data 410) are fed as input for a supervised learning algorithm 420 ("Model 1"). The algorithm 420 predicts the best possible browsing path 425 (along with the suitable solution) that can solve the fault in an efficient way consuming minimal time. Another model 430 ("Model 2"), with input as the fault description 410 and browsing trails 440, predicts the next best step 450 (i.e. suggest next log file) in the browsing path in view of the previous steps taken (e.g. preceding log files reviewed), ensuring the fastest route to solution. Each of the mentioned models comprises a GNN model.

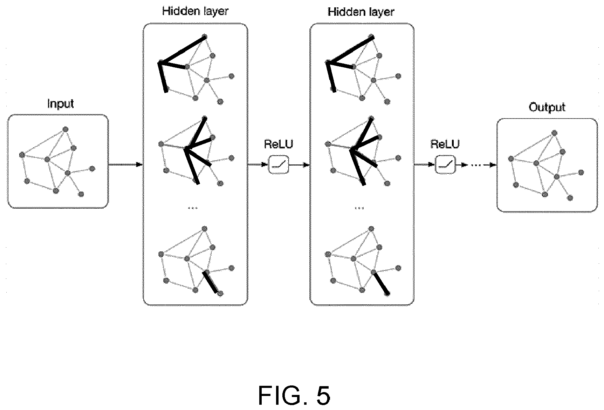

Fig. 5 depicts an exemplary structure of a GNN that may be employed by each of the models. For a given graph (with respect to a browsing trail), the GNN model is configured to predict the link with the next best step (i.e. log file) which is one of the candidate node. The GNN models the relationship, context and connections among the browsing trails graph nodes. The model is trained on previous log file browsing path graphs and learns different features of each node, state of the neighbouring node and features of neighbouring node to predict the next best step (i.e. next log file), the link to the best fitted node.

Explainable AI (XAI) can be used to highlight the important words responsible for the prediction. This helps is gaining confidence of the service engineer FSE as the highlighted words gives an idea as to why the browsing trial and the solution was chosen for a particular problem statement.

If the service engineer agrees to the output, a higher weightage can be provided to the predicted output, and is further used with high priority for predicting solution to similar problem presented.

To determine the best visualization, visualization steps may be determined by analysing parameters of the predicted/identified next step. For instance, if the next step is to look at the temperature of a parameter, then it is a numeric value over a period in Celsius or Fahrenheit. A time series plot can be created and shown along with a regression line or other statistical measure.
- If the next step involves a two parameters or same time for distinguishing abnormal from normal, then a scatter plot of the two variables may be employed.
- If the next step involves reviewing textual information in an error list, then a table can be shown with associated event times.

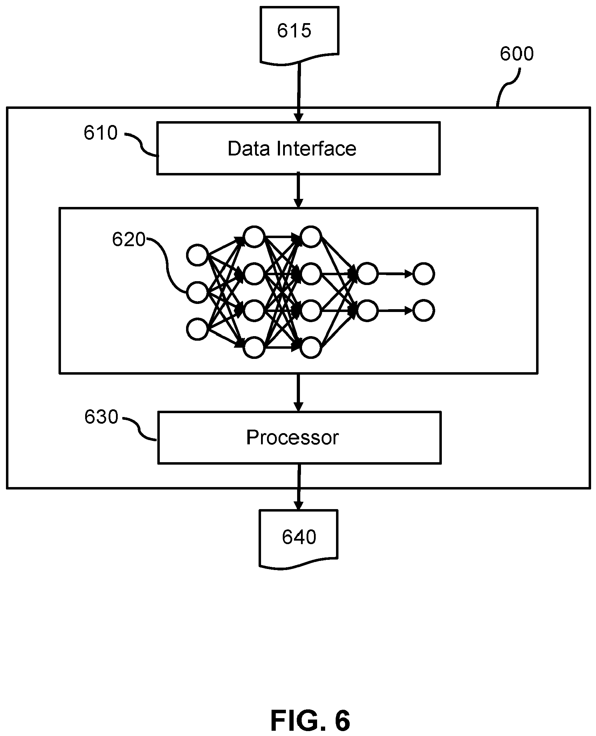

By the summary of a proposed implementation, Fig. 6 depicts a simplified block diagram of a system 600 for identifying a log file for resolution of a fault of a medical imaging device.

The system comprises a data interface 610 that is configured to obtain log file browsing data describing one or more log files of the medical imaging device already viewed by a user to resolve the fault of the medical imaging device; and problem data describing the fault of the medical imaging device. As detailed above, the problem input data may comprise textual description of the fault provided by a user.

A machine learning algorithm 620 of the system 600 is configured to receive the problem data and the log file browsing data. The machine learning algorithm 630 is trained to output a prediction result comprising a resolution probability for each of a plurality of log files of the medical imaging device, based on the browsing data. Here, a resolution probability indicates a likelihood that the log file will assist in resolution of the fault of the medical imaging device.

The system 600 also comprises a processor 630 that is configured to identify a log file for resolution of the fault of the medical imaging device based on the obtained prediction result. The identified log file is provided as an output 640 from the system 600, and may thus be used for prioritising the provision of log files to a user (e.g. remote service engineer).

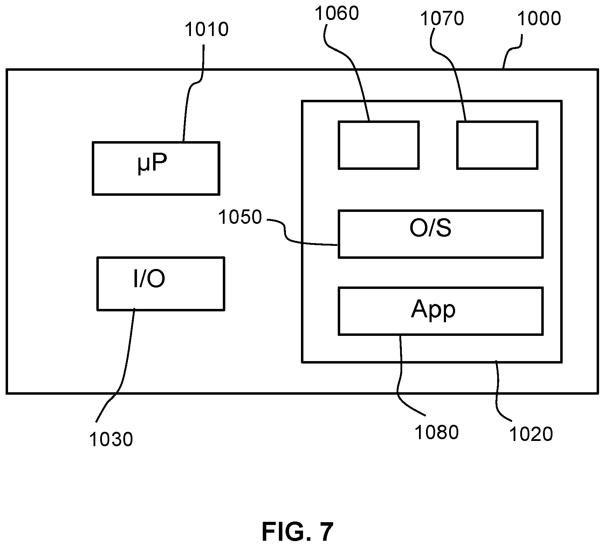

Fig. 7 illustrates an example of a computer 1000 within which one or more parts of an embodiment may be employed. Various operations discussed above may utilize the capabilities of the computer 1000. For example, one or more parts of a system for identifying a log file for resolution of a fault of a medical imaging device may be incorporated in any element, module, application, and/or component discussed herein. In this regard, it is to be understood that system functional blocks can run on a single computer or may be distributed over several computers and locations (e.g. connected via internet).

The computer 1000 includes, but is not limited to, PCs, workstations, laptops, PDAs, palm devices, servers, storages, and the like. Generally, in terms of hardware architecture, the computer 1000 may include one or more processors 1010, memory 1020 and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), or an auxiliary processor among several processors associated with the computer 1000, and the processor 1010 may be a semiconductor based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 1020 may incorporate electronic, magnetic, optical, and/or other types of storage media. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. It is contemplated by the inventors that the application 1080 for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 1030 may include input devices such as, for example but not limited to, a mouse, keyboard, scanner, microphone, camera, etc. Furthermore, the I/O devices 1030 may also include output devices, for example but not limited to a printer, display, etc. Finally, the I/O devices 1030 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of read-only-memory, such as ROM, PROM, EPROM, EEPROM or the like, so that the BIOS can be executed when the computer 800 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 1010, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-3 and the systems of Figs. 4-6, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Figs. 4-6 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method for identifying a log file for resolution of a fault of a medical imaging device, the method comprising:
obtaining (110) log file browsing data describing one or more log files of the medical imaging device already viewed by a user to resolve the fault of the medical imaging device;
obtaining (120) problem data describing the fault of the medical imaging device;
inputting (130) the problem data and the log file browsing data to a machine learning algorithm, the machine learning algorithm being trained to predict, for each of a plurality of log files of the medical imaging device, and based on the browsing data, a resolution probability indicating a likelihood that the log file will assist in resolution of the fault of the medical imaging device;
obtaining (140) a prediction result from the machine learning algorithm in response to the inputting, the prediction result comprising a resolution probability for one or more of the plurality of log files of the medical imaging device; and
identifying (150) a log file for resolution of the fault of the medical imaging device based on the obtained prediction result.

2. The method of claim 1, wherein the machine learning algorithm is trained using a training algorithm configured to receive an array of training inputs and respective known outputs, wherein a training input comprises: problem data; and log file browsing data, and wherein a respective known output comprises, for each of a plurality of log files of the medical imaging device, a resolution probability indicating a likelihood that the log file will assist in resolution of the fault of the medical imaging device based on the browsing data,
and optionally wherein a respective known output further comprises a description of solution associated with the problem data.

3. The method of claim 1 or 2, wherein obtaining (110) log file browsing data comprises:
monitoring access to the plurality of log files of the medical imaging device of file access; and
generating log file browsing data based on the result of monitoring.

4. The method of any of claims 1 to 3, wherein (110) obtaining log file browsing data comprises:
analysing a usage history of a log file viewer application.

5. The method of any of claims 1 to 4, wherein obtaining problem input data comprises:
receiving, via in input interface, problem data provided by a respondent in response to a request for data,
and optionally wherein the request for data comprises a fault analysis questionnaire.

6. The method of any of claims 1 to 5, wherein obtaining problem data describing the fault of the medical imaging device comprises:
performing (120) a natural language processing analysis on a description of the fault of the medical imaging device.

7. The method of any of claims 1 to 6, further comprising transmitting the identified log file over a communication network.

8. The method of any of claims 1 to 6, further comprising:
analysing the identified log file to provide an indication of the fault of the medical imaging device, and optionally adjusting one or more operational parameters of the medical imaging device to resolve the fault of the medical imaging device.

9. A method for resolving a fault of a medical imaging device, the method comprising:
identifying a log file for resolution of the fault of the medical imaging device according to the method of any of claims 1 to 8;
generating updated log file browsing data based on the identified log file;
inputting (130) the problem data and the updated log file browsing data to the machine learning algorithm;
obtaining (140) an updated prediction result from the machine learning algorithm in response to the inputting, the updated prediction result comprising a resolution probability for one or more of the log files of the medical imaging device; and
identifying (150) a second log file for resolution of the fault of the medical imaging device based on the obtained prediction result.

10. A computer program comprising code means for implementing the method of any preceding claim when said program is run on a processing system.

11. A system for identifying a log file for resolution of a fault of a medical imaging device, the system comprising:
a data interface (910) configured to obtain: log file browsing data describing one or more log files of the medical imaging device already viewed by a user to resolve the fault of the medical imaging device; and problem data describing the fault of the medical imaging device;
a machine learning algorithm (930) configured to receive the problem data and the log file browsing data, the machine learning algorithm being trained to output a prediction result comprising a resolution probability for each of a plurality of log files of the medical imaging device, the resolution probability indicating a likelihood that the log file will assist in resolution of the fault of the medical imaging device based on the browsing data; and
a processor (940) configured to identify a log file for resolution of the fault of the medical imaging device based on the obtained prediction result.

12. The system of claim 11, wherein the data interface comprises a monitoring component adapted to monitor access to the plurality of log files of the medical imaging device of file access and to generate log file browsing data based on the result of monitoring.

13. The system of claim 11 or 12, wherein the data interface comprises a natural language processor configured to performing a natural language processing analysis on a description the fault of the medical imaging device.

14. The system of any of claims 11 to 13, wherein the machine learning algorithm comprises a graph neural network.

15. A system for resolving a fault of a medical imaging device, the method comprising:
the system for identifying a log file for resolution of the fault of the medical imaging device according to any of claims 11 to 14;
a data processor configured to generate updated log file browsing data based on the identified log file;
wherein the machine learning algorithm is configured to receive the problem data and the updated log file browsing data and to output an updated prediction result, the updated prediction result comprising a resolution probability for one or more of the log files of the medical imaging device,
and wherein the processor is configured to identify a second log file for resolution of the fault of the medical imaging device based on the obtained prediction result.
